# EUROPÄISCHE PATENTANMELDUNG

(11) **EP 1 842 566 A2**
(43) Veröffentlichungstag der Anmeldung: **10.10.2007**
(21) Anmeldenummer: 07005648.6
(22) Anmeldetag: 20.03.2007
(51) Int. Cl.: A61L 27/34

(54) **Ausstattung von Vaskulärimplantaten mit einem Wirkprinzip**

(30) Priorität: 06.04.2006 DE 102006016598
(71) Anmelder: Heraeus Kulzer GmbH, 63450 Hanau (DE)
(72) Erfinder: Kühn, Klaus-Dieter, Dr., 35041 Marburg-Elnhausen (DE); Vogt, Sebastian, Dr., 99092 Erfurt (DE); Schnabelrauch, Matthias, Dr., 07749 Jena (DE)
(74) Vertreter: Kühn, Hans-Christian

(57) **Zusammenfassung**

Ein oder mehrere Stoffe der Gruppe bestehend aus Gentamicinpalmitat, Gentamicinmyristat, Gentamicinlaurat, Tobramycinpalmitat, Tobramycinmyristat, Tobramycinlaurat, Amikacinpalmitat, Amikacinmyristat, Amikacinlaurat, Vancomycinpalmitat, Vancomycinlaurat und Vancomycinmyristat sind zur antithrombogenen Ausstattung von Vaskulärimplantaten geeignet. Ein entsprechendes Verfahren zur Ausstattung von Vaskulärimplantaten mit einem antithrombogenen Wirkprinzip besteht insbesondere darin, dass der Implantatkörper
A in eine alkoholische Lösung oder eine alkoholische Lösung mit einem leicht flüchtigen Lösungsmittel, wie z.B. Chloroform,
eines Mitglieds der Gruppe des Gentamicinpalmitats, des Gentamicinmyristats, des Gentamicinlaurats, des Tobramycinpalmitats, des Tobramycinmyristats, des Tobramycinlaurats, des Amikacinpalmitats, des Amikacinmyristats, des Amikacinlaurats, des Vancomycinpalmitats und des Vanocmycinmyristats
getaucht wird oder mit dieser Lösung besprüht wird,
B und das alkoholische Lösungsmittel verdampft wird.

## Beschreibung

Gegenstand der Erfindung ist die Ausstattung von Vaskulärimplantaten mit einem Wirkprinzip.

Gefäßprothesen werden gegenwärtig in großem Umfang in der Gefäßchirurgie bei vaskulären Defekten eingesetzt. Dabei kommen insbesondere poröse PTFE-Prothesen und gestrickte Prothesen aus Polyester (DACRON) zur Anwendung. Nach Implantation der Gefäßprothesen kann es besonders in den ersten Stunden nach dem Wiedereinsetzen des Blutstromes zu einer Thrombenbildung im Bereich der Gefäßprothese kommen, wodurch der Blutstrom behindert bzw. unterbrochen wird und der gebildete Thrombus kann durch Bakterien besiedelt werden. Die Problematik infizierter Gefäßprothesen ist auch im Zeitalter moderner Antibiotika eine gefürchtete Nebenwirkung und stellt für den Patienten eine lebensbedrohliche Gefährdung dar. Sie kann zum Verlust des gefäßführenden Organs/Sepsis und in deren Folge einen septischen Schock verursachen, der zum Tod des Patienten führen kann.

Es ist daher wünschenswert, Vaskulärimplantate antithrombogen zu beschichten, um besonders in den ersten Stunden nach der Implantation eine Thrombenbildung im Implantatbereich wirksam zu verhindern, bevor die Endothelisierung der Implantatoberfläche einsetzt.

Antithrombogene Beschichtungen auf Basis von Heparin, Heparinderivaten und sulfatierten Polyssachariden sowie sulfatierten Polysacharidderivaten sind aus einer Vielzahl von Patentanmeldungen bekannt, z.B. aus CA 2510220 A1; US 2006014720 A1 oder W02005118018 A1.

Der Erfindung liegt die Aufgabe zu Grunde, eine Beschichtung für Vaskulärimplantate bereit zu stellen, die über einen Zeitraum von mehreren Stunden eine antithrombogene auf der porösen oder auch geschlossenen Oberfläche von Vaskulärimplantaten in Gegenwart des menschlichen Blutstroms ausüben kann.

Die Aufgabe wird durch die Verwendung von einem oder mehreren Stoffen der Gruppe bestehend aus Gentamicinpalmitat, Gentamicinmyristat, Gentamicinlaurat, Tobramycinpalmitat, Tobramycinmyristat, Tobramycinlaurat, Amikacinpalmitat, Amikacinmyristat, Amikacinlaurat, Vancomycinpalmitat, Vancomycinlaurat und Vancomycinmyristat zur antithrombogenen Ausstattung von Vaskulärimplantaten gelöst.

Der Erfindung liegt die überraschende Beobachtung zu Grunde, dass die an sich bekannten in Wasser gering löslichen Fettsäuresalze der Aminoglykosid-Antibiotika eine ausgeprägte antithrombogene Wirkung zeigen.

Gemäß der Erfindung können z.B. poröse PTFE- oder Polyester-Gefäß-Prothesen mit Fettsäuresalzen der Amino-glykosid-Antibiotika so beschichtet werden, dass die Beschichtung auf dem PTFE haftet unter Erhalt der offen porösen Struktur. Überraschend ist, dass bei den beschichteten Gefäß-Prothesen die notwendige Geschmeidigkeit erhalten bleibt, ohne dass eine Ablösung der Beschichtung erfolgt.

Die Erfindung betrifft entsprechend auch Verfahren zur Ausstattung von Vaskulärimplantaten mit einem antithrombogenen Wirkprinzip, wie weiter unten beschrieben.

In die erfindungsgemäß eingesetzten Fettsäuresalze der Aminoglykosid-Antibiotika können optional weitere Inhibitoren der Blutgerinnung und/oder der Plättchen-aggregation sowie auch DNA oder RNA oder synthetische Analoga der DNA suspendiert oder molekulardispers eingearbeitet werden, ohne die schichtbildenden Eigenschaften der Fettsäuresalze zu verändern.

Solche weiteren synthetischen oder natürlichen Inhibitoren der Blutgerinnung und/oder der Plättchenaggregation und/ oder die offenkettige oder cyclische DNA oder RNA oder die synthetische DNA-Analoga und/oder die Addukte aus offenkettiger oder cyclischer DNA oder RNA oder synthetischer Analoga der DNA und einem oder mehreren kationischen Antibiotika sind in der Beschichtung teilweise oder vollständig eingeschlossen. Als kationische Antibiotika sind hierbei Aminoglykosid-Antibiotika, Lincosamid-Antibiotika und Chinolonantibiotika geeignet. Dabei sind Gentamicin, Amikacin, Tobramycin, Clindamycin, Lincosamin, Ofloxacin und Moxifloxacin besonders bevorzugt.

Es kann ein weiterer Arzneistoff in der Beschichtung dispergiert sein, wobei der Arzneistoff auch molekulardispers in der Beschichtung enthalten sein kann.

Als antithrombogene Wirkstoffe kommen insbesondere Argatroban, Heparin und synthetisch gewonnene Polysaccharidsulfate in Frage.

Die gegebenenfalls eingesetzte offenkettige oder cyclische DNA oder RNA oder deren synthetische Analoga kodieren bevorzugt Wachstumsfaktoren oder Angiogenesefaktoren.

Dabei wirken die Fettsäuresalze der Aminoglykosid-Antibiotika gleichzeitig antithrombogen und als schichtbildende Stoffe.

Das erfindungsgemäße Verfahren zur Ausstattung von Vaskulärimplantaten mit einem antithrombogenen Wirkprinzip besteht insbesondere darin, dass der Implantatkörper
A in eine alkoholische Lösung oder eine alkoholische Lösung mit einem leicht flüchtigen Lösungsmittel, wie z.B. Chloroform,
   eines Mitglieds der Gruppe des Gentamicinpalmitats, des Gentamicinmyristats, des Gentamicinlaurats, des Tobramycinpalmitats, des Tobramycinmyristats, des Tobramycinlaurats, des Amikacinpalmitats, des Amikacinmyristats, des Amikacinlaurats, des Vancomycinpalmitats und des Vanocmycinmyristats
   getaucht wird oder mit dieser Lösung besprüht wird,
B und das alkoholische Lösungsmittel verdampft wird.

Der Implantatkörper kann auch mit einer alkoholischen Lösung eines Mitglieds der Gruppe bestehend aus Gentamicinpalmitat, Gentamicinmyristat, Gentamicinlaurat, Tobramycinpalmitat, Tobramycinmyristat, Tobramycinlaurat, Amikacinpalmitat, Amikacinmyristat, Amikacinlaurat, Vancomycinpalmitat, Vancomycinlaurat und Vancomycinmyristat besprüht werden, in der ein synthetischer oder natürlicher Inhibitor der Blutgerinnung und/oder der Blättchenaggregation und/oder eine offenkettige oder cyclische DNA oder RNA oder ein synthetisches Analoga der DNA und/oder ein oder mehrere Addukte, die aus offenkettiger oder cyclischer DNA oder RNA oder synthetischer Analoga der DNA und einem oder mehreren kationischen Antibiotika aufgebaut sind, suspendiert sind, wobei wie vorher die Beschichtung durch Verdampfen des alkoholischen Lösungsmittels ausgebildet wird.

Die resultierende Schicht ist vorzugsweise 0,1 µm bis 200 µm dick.

Im Falle von PTFE oder Polyester als Gefäßprothesenmaterial ist es zweckmäßig, wenn die Schicht die vorhandenen Porensysteme nicht vollständig verschließt.

Die Erfindung wird durch nachstehende Beispiele erläutert, ohne jedoch die Erfindung zu beschränken. Teile- und Prozentangaben beziehen sich auf das Gewicht, sofern nicht anders angegeben.

### Beispiel 1:

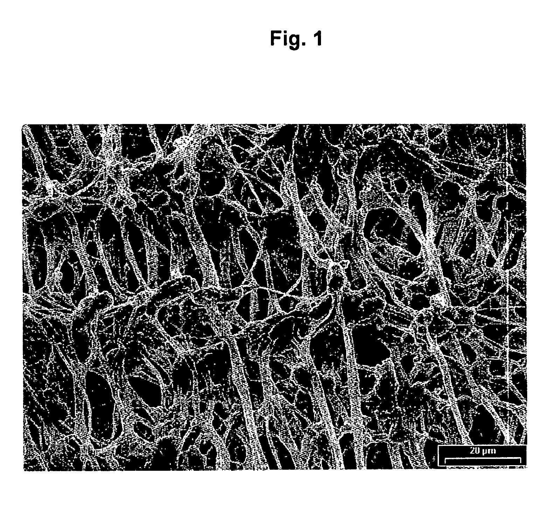

Es wurde eine gereckte PTFE-Gefäß-Prothese (Durchmesser 6 mm) in eine 5 Ma%ige methanolische Lösung von Gentamicinpalmitat bei Raumtemperatur für 60 Sekunden getaucht. Danach wurde die PTFE-Gefäß-Prothese bei Raumtemperatur bis zur Massekonstanz getrocknet. Die aufgebrachte Gentamicinpalmitatschicht wurde gravimetrisch erfasst. Es wurde eine Beladung von 0,95 mg Gentamicinpalmitat pro Zentimeter der PTFE-Gefäß-Prothese gefunden. Eine rasterelektronenmikroskopische Aufnahme der beschichteten PTFE-Gefäß-Prothese ist in Fig. 1 dargestellt.

### Beispiel 2:

Es wurde eine gereckte PTFE-Gefäß-Prothese (Durchmesser 6 mm) in eine 5 Ma%ige methanolische Lösung von Gentamicinpalmitat, die 1 Ma% Argatroban enthält, bei Raumtemperatur für 60 Sekunden getaucht. Danach wurde die PTFE-Gefäß-Prothese bei Raumtemperatur bis zur Massekonstanz getrocknet. Die aufgebrachte Gentamicinpalmitatschicht wurde gravimetrisch erfasst. Es wurde eine Beladung von 0,97 mg pro Zentimeter gefunden.

## Patentansprüche

1. Verfahren zur Ausstattung von Vaskulärimplantaten mit einem Wirkprinzip,
wobei
der Implantatkörper
A in eine alkoholische Lösung oder eine alkoholische Lösung mit einem leicht flüchtigen Lösungsmittel, wie z.B. Chloroform,
eines Mitglieds der Gruppe des Gentamicinpalmitats, des Gentamicinmyristats, des Gentamicinlaurats, des Tobramycinpalmitats, des Tobramycinmyristats, des Tobramycinlaurats, des Amikacinpalmitats, des Amikacinmyristats, des Amikacinlaurats, des Vancomycinpalmitats und des Vanocmycinmyristats
getaucht wird oder mit dieser Lösung besprüht wird,
B und das alkoholische Lösungsmittel verdampft wird,
**dadurch gekennzeichnet, dass** das Wirkprinzip antithrombogen ist.

2. Verfahren nach Anspruch 1, wobei in der Lösung des Schritts A zusätzlich
• ein synthetischer oder natürlicher Inhibitor(s) der Blutgerinnung und/oder der Plättchenaggregation oder
• eine offenkettige oder cyclische DNA oder RNA oder ein synthetisches Analogon der DNA und/oder ein oder mehrere Addukte, die aus offenkettiger oder cyclischer DNA oder RNA oder synthetischer Analoga der DNA und einem oder mehreren kationischen Antibiotika aufgebaut sind,
suspendiert ist/sind.

3. Beschichtete Vaskulärimplantate, hergestellt nach einem Verfahren des Anspruchs 1 oder 2, **dadurch gekennzeichnet, dass** ein weiterer Arzneistoff in der Beschichtung dispergiert oder suspendiert ist.

4. Beschichtete Vaskulärimplantate nach mindestens Anspruch 3, wobei der Arzneistoff molekulardispers in der Beschichtung enthalten ist.

5. Beschichtete Vaskulärimplantate nach Anspruch 3 oder 4, **dadurch gekennzeichnet, dass** als antithrombogene(r) Wirkstoff(e) mindestens ein Mitglied der Gruppe bestehend aus Argatroban, Heparin und synthetisch gewonnenen Polysaccharidsulfaten eingesetzt wird.

6. Beschichtete Vaskulärimplantate nach mindestens einem der Ansprüche 3 bis 5, **dadurch gekennzeichnet, dass** die offenkettige oder cyclische DNA oder RNA oder deren synthetische Analoga Wachstumsfaktoren oder Angiogenesefaktoren kodiert.

7. Beschichtete Vaskulärimplantate nach mindestens einem der Ansprüche 3 bis 6, **dadurch gekennzeichnet, dass** die Schicht 0,1 µm bis 200 µm dick ist.

8. Beschichtete Vaskulärimplantate nach mindestens einem der Ansprüche 3 bis 7, wobei der Implantatkörper aus porösem PTFE oder Polyester besteht.

9. Beschichtete Vaskulärimplantate nach Anspruch 8, wobei die Schicht die Porensysteme nicht vollständig verschließt.

10. Verwendung von Verbindungen der Gruppe bestehend aus Gentamicinpalmitat, Gentamicinmyristat, Gentamicinlaurat, Tobramycinpalmitat, Tobramycinmyristat, Tobramycinlaurat, Amikacinpalmitat, Amikacinmyristat, Amikacinlaurat, Vancomycinpalmitat und Vanocmycinmyristat zur Ausstattung von Vaskulärimplantaten mit einem antithrombogenen Wirkprinzip.
